**Europäisches Patentamt**

**European Patent Office**

**Office européen des brevets**

(11) Veröffentlichungsnummer:

**0 035 140**
A1

(12) # EUROPÄISCHE PATENTANMELDUNG

(21) Anmeldenummer: 81100950.5

(22) Anmeldetag: 11.02.81

(51) Int. Cl.³: **G 01 N 33/34**, G 01 N 23/06, G 01 N 23/203

(30) Priorität: **27.02.80 DE 3007328**

(43) Veröffentlichungstag der Anmeldung: **09.09.81** Patentblatt 81/36

(84) Benannte Vertragsstaaten: **DE FR SE**

(71) Anmelder: **BASF Aktiengesellschaft, Carl-Bosch-Strasse 38, D-6700 Ludwigshafen (DE)**

(72) Erfinder: **Melzer, Jaroslav, Kirchenstrasse 116, D-6700 Ludwigshafen (DE)**
Erfinder: **Troester, Robert, In den Mooraeckern 7, D-6700 Ludwigshafen (DE)**
Erfinder: **Weiss, Gregor, Blockfeldstrasse 38, D-6704 Mutterstadt (DE)**

(54) **Messeinrichtung zur Flächen- und Füllstoffgewichtsbestimmung von Papierproben.**

(57) Meßeinrichtung zur Bestimmung des Flächen- und Füllstoffgewichts von Papierproben (7) mit einer auf der Absorption bzw. Rückstreuung von Betastrahlen beruhenden Meßanordnung (8, 9) und einer daran vorbeibewegbaren Scheibe (1) mit Aufnahmen (6) für die Papierproben (7).

EP 0 035 140 A1

BASF Aktiengesellschaft

C035140
O.Z. 0050/034316

Meßeinrichtung zur Flächen- und Füllstoffgewichtsbestimmung von Papierproben

Die Erfindung betrifft eine Meßeinrichtung zur Bestimmung des Flächen- und Füllstoffgewichts von Papierproben unter Anwendung von Radioisotopen.

Es ist bereits bekannt, kontinuierliche Flächen- und Füllstoffgewichtsmessungen mit Betastrahlen an laufenden Papierbahnen durchzuführen. Die Arbeitsweise entsprechender Meßanlagen beruht, wie beispielsweise in einem Sonderdruck aus der Fachzeitschrift "Das Papier", Ausgabe 12, Dezember 1970, Seite 937-941; "Kontinuierliche Füllstoffmessung mit Betastrahlen an laufenden Papierbahnen" von Ing. Wilhelm Carl, und in einem Sonderdruck aus der Fachzeitschrift "Kerntechnik Isotopentechnik und -Chemie" 5. Jg. 1963, Heft 10, Seite 411-417: "Flächengewichtsregelung unter Anwendung von Radioisotopen" von H. Langel beschrieben, auf der Absorption und Rückstreuung der Betastrahlen von Radioisotopen im Papier. Dabei werden die Schwächung der das Papier durchdringenden Strahlung als Maß für das Flächengewicht und die Intensität der Rückstreustrahlung als Maß für das Füllgewicht benutzt.

Während derartige Meßanlagen der direkten Überwachung der Papierherstellung dienen, wurden für die Untersuchung von Papierproben in Entwicklungs- und Prüflabors die Gewichtsbestimmungen bisher über das Veraschen des Papiers durchgeführt. In einem Veraschungsapparat wird die Papierprobe verbrannt und darauf folgend geglüht. Die zurückbleibende Asche wird gewogen und auf das Gewicht des Papiers bezogen. Nach Abzug des Glühverlustes des jeweiligen Füllstoffes stellt das Ergebnis den Füllstoffanteil, beispielsweise aus Kaolin, Kreide oder Titandioxid, dar.

Sp/BL

Die Messung erfordert viele zeitraubende Arbeitsschritte, die von Hand ausgeführt werden müssen und Rechenoperationen beinhalten. Dadurch sind Fehlerquellen, z.B. durch das Ablesen bei der Gewichtsbestimmung und Umrechnen der Meßergebnisse, gegeben. Ferner ist die bisherige Meßmethode für große, immer wiederkehrende Meßreihen nicht leistungsfähig genug.

Es stellte sich daher die Aufgabe, eine Meßeinrichtung zu entwickeln, bei der die Flächen- und Füllstoffgewichtsbestimmung von Papierproben ebenfalls mit Hilfe von Radioisotopen durchgeführt werden kann, um einen weitgehend automatischen und fehlerfreien Meßablauf zu erhalten.

Die Aufgabe wurde durch eine Meßeinrichtung gelöst, die gekennzeichnet ist durch eine drehbar gelagerte Scheibe, die mit mehreren Aufnahmen für die Papierproben ausgestattet und in deren Anzahl entsprechenden Schritten motorisch antreibbar ist, und durch zwei Positionen der Aufnahmen bezüglich der Drehschnitte, denen jeweils eine an sich bekannte, auf der Absorption bzw. Rückstreuung von Betastrahlen beruhende Meßanordnung zugeordnet ist.

In einer vorteilhaften Ausführungsform der Meßeinrichtung bestehen die Aufnahmen aus Ringen, die an ihrem Innenrand eine Abstufung für die Auflage der Papierproben aufweisen und in kreisrunden Ausschnitten der Scheibe drehbar gehalten und in den Meßanordnungen zugeordneten Positionen mit einem motorischen Antrieb in Verbindung bringbar sind.

Bei einer bevorzugten Ausführungsform der Meßeinrichtung ist der motorische Antrieb durch einen Elektromotor und durch wenigstens ein mit dessen Rotorwelle verbundenen Reibrad gebildet, das an einer der Ringflächen angreift.

In Weiterbildung der Erfindung ist der Scheibe mit den Aufnahmen eine positionierbare und pneumatisch betätigbare Entnahme- und Ablegevorrichtung für die Papierproben zugeordnet.

Die erfindungsgemäße Meßeinrichtung gewährleistet bei wesentlich schnellerer Durchführung der Messungen Ergebnisse hoher Genauigkeit und guter Vergleichbarkeit, wobei die bisherigen Fehlerquellen ausgeschaltet sind. Weiterhin besteht die Möglichkeit, die Meßergebnisse durch ein beigefügtes Rechenwerk oder den Anschluß an einen Prozeßrechner auszuwerten.

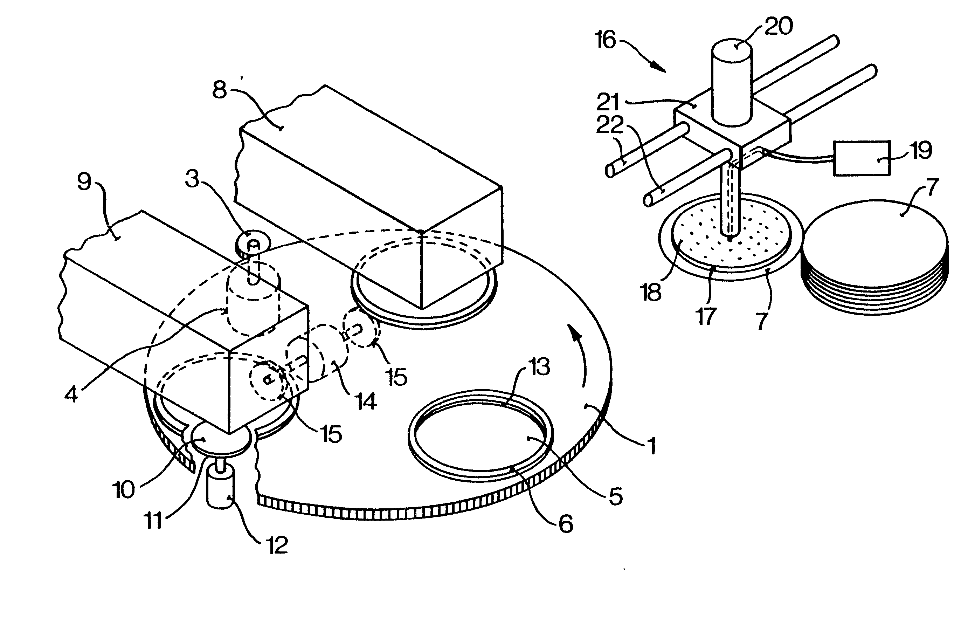

Weitere Vorteile und Einzelheiten ergeben sich aus der nachfolgenden Beschreibung eines in der Zeichnung dargestellten Ausführungsbeispiels.

In der Zeichnung ist die erfindungsgemäße Meßeinrichtung in perspektivischer Gesamtansicht schematisch wiedergegeben.

Eine Scheibe 1 ist drehbar gelagert und über ein Getriebe 3 von einem Elektromotor 4 antreibbar. Ferner weist sie drei entlang des Scheibenumfangs gleichmäßig verteilte, kreisrunde Ausschnitte 5 auf, in denen Ringe 6 zur Aufnahme der scheibenförmigen Papierproben 7 mittels Kugeln drehbar gelagert sind. Am Innenrand besitzen die Ringe eine Abstufung 13 als Auflagefläche für die Papierproben.

Durch Drehen der Scheibe 1 in Schritten entsprechend der durch die Anzahl der Ringe gegebenen Teilung der Scheibe – beim vorliegenden Ausführungsbeispiel um jeweils 120° – gelangen die Papierproben in die einzelnen Positionen für das Einlegen bzw. für die Entnahme der Proben und die Messungen. Zwei dieser Positionen ist jeweils eine Meß-

C035140 O.Z. 0050/034316

apparatur mit einem Radioisotop und einem Strahlendetektor der bekannten, eingangs beschriebenen Art zugeordnet. Bei der Meßapparatur 8 für die Flächengewichtsmessung befindet sich der Strahlerhalter mit dem Radioisotop über der Papierprobe und der zugehörige Strahlendetektor für die Durchstrahlmessung unterhalb der Probe. Für die Füllstoffgewichtsmessung bei der Meßapparatur 9 befindet sich der Detektor zur Messung der Strahlenrückstreuung über der Papierprobe, wobei unterhalb derer ein Reflektor 10 aus Zellstoff oder Plexiglas mit Aluminium angeordnet ist, getragen von einer Aufnahmeplatte 11, die mit Hilfe eines pneumatischen Arbeitszylinders 12 unmittelbar an die Papierprobe fahrbar ist. Wirkungsweise und technischer Aufbau der Meßapparaturen sind ausführlich in der Fachliteratur, insbesondere in den eingangs erwähnten Fachzeitschriften, beschrieben, so daß weiters nicht näher auf sie eingegangen zu werden braucht. In der Zeichnung sind sie daher auch lediglich durch die Meßapparaturgehäuse mit Bezugszeichen 8 und 9 angedeutet.

Zur Erfassung einer möglichst großen Fläche der zu messenden Papierproben 7 ist es zweckmäßig, die Ringe 6 mit den Proben bei den Meßapparaturen 8 und 9 während der Messungen rotieren zu lassen. Die Umdrehungszahl liegt vorteilhaft zwischen 20 und 100 U/min. Der Antrieb erfolgt von einem unterhalb der Scheibe 1 fest montierten Elektromotor 14 über Reibräder 15 an einer der Ringflächen.

Die Entnahme der Papierproben 7 aus den Ringen 6 sowie das Einlegen in diese kann ebenfalls mechanisiert werden, indem der Scheibe 1 eine positionierbare und pneumatisch betätigbare Entnahme- und Ablagevorrichtung 16 zugeordnet wird. Diese besteht in der bevorzugten Ausführungsform der Meßeinrichtung aus einem Teller 17, der zum Aufnehmen, Festhalten und Ablegen jeweils einer Papierprobe mit Saug-

C035140

Öffnungen 18 versehen ist, die mit einer Unterdruckquelle 19 in Verbindung gebracht werden können. Der Teller ist mit Hilfe eines pneumatischen Arbeitszylinders 20 heb- und senkbar, und dieser wiederum mit einer Trägerplatte 21 auf Schienen 22 zu der entsprechenden Ringposition fahrbar. Der Antrieb der Trägerplatte erfolgt ebenfalls durch einen pneumatischen Arbeitszylinder (in der Zeichnung nicht dargestellt).

Mit Hilfe einer elektronischen Steuerung kann der gesamte Meßablauf automatisiert werden. Geeignete Steuerungen können im Fachhandel bezogen werden und sind von einschlägigen Fachkräften an die vorliegende Anwendung anpaßbar, so daß auf die Beschreibung einer solchen Einrichtung verzichtet werden kann.

Der Meßablauf beginnt mit dem Einlegen einer Papierprobe 7 von einem Vorratsstapel in den freiliegenden Ring 6 mit Hilfe der Vorrichtung 16. Dabei kann die zu prüfende Stückzahl der Proben an einem Vorwahlzähler der Steuerung eingestellt werden. Nach dem Einlegen der Papierprobe gelangt diese durch einen Drehschritt der Scheibe 1 von $120^{\circ}$ unter die Meßapparatur 8 für die Flächengewichtsbestimmung. In dieser Stellung der Scheibe wird bereits die nächste Papierprobe in den nachfolgenden Ring 6 eingelegt. Das Meßergebnis wird in ein an die Meßapparaturen angeschlossenes Rechenwerk übertragen und dort zunächst gespeichert. Ein weiterer Drehschritt von $120^{\circ}$ bringt die Papierprobe unter die Meßapparatur 9 für die Füllstoffgewichtsbestimmung und die nachfolgende Probe zur Apparatur 8. Während der beiden Messungen werden die Ringe 6 durch den Motor 14 über die Reibräder 15 in Drehung versetzt. Vor Beginn der Füllstoffgewichtsmessung wird die Aufnahmeplatte 11 mit dem Reflektor 10 mittels des pneumatischen Arbeitszylinders 12 unmittelbar an die Papierprobe 7 gefahren und nach der

C035140

Messung wieder entfernt, damit die Scheibe 1 weitergedreht werden kann. Das Meßergebnis wird wiederum im Rechenwerk gespeichert, welches anschließend durch die Verhältnisbildung von Füllstoffgewicht zu Flächengewicht die relative Füllstoffmenge in Prozent errechnet und ein diesem Wert entsprechendes elektrisches Signal bildet, das zu einer in der Zeichnung nicht dargestellten Anzeigeeinrichtung - beispielsweise zu einem Zeigerinstrument oder einer digitalen Anzeige oder aber zu einem Schreiber - übertragen wird. Nach einem weiteren Drehschritt der Scheibe von 120° steht der Ring wieder in der Ausgangsposition, so daß die gemessene Papierprobe durch die Vorrichtung 16 entfernt und eine neue Probe eingelegt werden kann. Selbstverständlich kann auch zum Entfernen der Probe eine geeignete Auswerfervorrichtung vorgesehen werden.

Versuche mit der erfindungsgemäßen Meßeinrichtung im technischen Maßstab haben den damit erreichbaren technischen Fortschritt demonstriert. In einer Stunde konnten ca. 120 Papierproben bearbeitet werden; für das gleiche Ergebnis mußte mit den bisher zur Verfügung stehenden Einrichtungen ein voller Arbeitstag aufgewendet werden.

C035140

## Patentansprüche

1. Meßeinrichtung zur Bestimmung des Flächen- und Füllstoffgewichts von Papierproben unter Anwendung von Radioisotopen, gekennzeichnet durch eine drehbar gelagerte Scheibe (1), die mit mehreren Aufnahmen (6) für die Papierproben (7) ausgestattet und in deren Anzahl entsprechenden Schritten motorisch antreibbar ist, und durch zwei Positionen der Aufnahmen bezüglich der Drehschritte, denen jeweils eine an sich bekannte, auf der Absorption bzw. Rückstreuung von Betastrahlen beruhende Meßanordnung (8, 9) zugeordnet ist.

2. Meßeinrichtung nach Anspruch 1, dadurch gekennzeichnet, daß die Aufnahmen aus Ringen (6) bestehen, die an ihrem Innenrand eine Abstufung (13) für die Auflage der Papierproben (7) aufweisen und in kreisrunden Ausschnitten (5) der Scheibe (1) drehbar gehalten und in den den Meßanordnungen (8, 9) zugeordneten Positionen mit einem motorischen Antrieb (14, 15) in Verbindung bringbar sind.

3. Meßeinrichtung nach Anspruch 2, dadurch gekennzeichnet, daß der motorische Antrieb durch einen Elektromotor (14) und durch wenigstens ein mit dessen Rotorwelle verbundenen Reibrad (15) gebildet ist, das an einer der Ringflächen angreift.

4. Meßeinrichtung nach Anspruch 1 bis 3, dadurch gekennzeichnet, daß der Scheibe (1) mit den Aufnahmen (6) eine positionierbare und pneumatisch betätigbare Entnahme- und Ablegevorrichtung (16) für die Papierproben (7) zugeordnet ist.

Zeichn.

0035140

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 3 525 865 (W.C. RUTLEDGE)<br>* Anspruch 1, Spalte 3, Zeilen 30 bis 50; Fig. 1 *<br>-- | 1 |
| | DE - A1 - 2 602 040 (CENTRE DE RECHER-CHES METALLURGIQUES)<br>* Ansprüche 1, 2, 4 *<br>-- | 1-3 |
| A | DE - A - 2 140 757 (ÖSTERREICHISCHE STUDIENGESELLSCHAFT FÜR ATOMENERGIE GMBH)<br>-- | |
| A | DE - A - 2 115 016 (MEASUREX CORP.)<br>-- | |
| A,D | KERNTECHNIK, Band 5, Nr. 10, 1963 München<br>H. LANGEL "Flächengewichtsregelung unter Anwendung von Radioisotopen" Seiten 411 bis 417<br>-- | |
| A,D | DAS PAPIER, Band 24, Nr. 12, 1970 W. CARL "Kontinuierliche Füllstoff-messung mit Betastrahlen an laufenden Papierbahnen" Seiten 937 bis 941<br>---- | |

**KLASSIFIKATION DER ANMELDUNG (Int. Cl.³)**

G 01 N 33/34
G 01 N 23/06
G 01 N 23/203

**RECHERCHIERTE SACHGEBIETE (Int. Cl.³)**

G 01 N 23/00
G 01 N 33/34

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 07-05-1981 | SCHWARTZ |

EPA form 1503.1 06.78